(19) Europäisches Patentamt
European Patent Office
Office européen des brevets

(11) Veröffentlichungsnummer: **0 219 457 B1**

(12) # EUROPÄISCHE PATENTSCHRIFT

(45) Veröffentlichungstag der Patentschrift: **11.01.89**

(51) Int. Cl.⁴: **C07C 65/10**, C07C 51/353, B41M 5/22, C07C 65/105, B41M 5/12

(21) Anmeldenummer: **86810424.1**

(22) Anmeldetag: **29.09.86**

(54) **Verfahren zur Herstellung von Metallsalzgemischen von ringsubstituierten Salicylsäureverbindungen.**

(30) Priorität: **03.10.85 CH 4286/85**

(43) Veröffentlichungstag der Anmeldung:
**22.04.87 Patentblatt 87/17**

(45) Bekanntmachung des Hinweises auf die Patenterteilung:
**11.01.89 Patentblatt 89/2**

(84) Benannte Vertragsstaaten:
**CH DE FR GB LI**

(56) Entgegenhaltungen:
**EP-A- 0 181 283**
**GB-A- 2 025 940**

**CHEMICAL ABSTRACTS, Band 95, Nr. 8, August 1981, Columbus, OH (US); B.-L.HE et al.: "Friedel-Craft reactions of the chloromethylated styrene-divinylbenzene copolymers", S. 46, Nr. 63331r**

(73) Patentinhaber: **CIBA-GEIGY AG, Klybeckstrasse 141, CH-4002 Basel(CH)**

(72) Erfinder: **Nachbur, Hermann, Mischelistrasse 21, CH-4153 Reinach(CH)**

EP 0 219 457 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft ein Verfahren zur Herstellung von Metallsalzgemischen von ringsubstituierten Salicylsäureverbindungen, welche Metallsalze als Farbentwickler für Farbbildner in druckempfindlichen oder wärmeempfindlichen Aufzeichnungsmaterialien Verwendung finden können.

Aus der DE-PS 2 242 250 sind Farbstoffakzeptoren oder Farbentwickler für Farbstoffvorläufer bekannt, die Salze eines mehrwertigen Metalles und einer Salicylsäureverbindung sind, wobei die Salicylsäureverbindung in 3- und 5-Stellung disubstituiert ist. Als derartige Substituenten der Salicylsäure werden beispielsweise α-Methylbenzyl, α,α-Dimethylbenzyl oder Cyclohexyl genannt. Gemäss der DE-PS 2 242 250 werden solche ringsubstituierten Salicylsäureverbindungen mit Hilfe substituierter Phenole und gasförmigem Kohlendioxid hergestellt, wobei die substituierten Phenole durch Umsetzung von Phenolen z.B. mit Styrol, α-Methylstyrol oder Cyclohexylchlorid erhalten werden.

Es wurde nun gefunden, dass man wirtschaftlich günstige Metallsalzgemische von ringsubstiuierten Salicylsäureverbindungen erhalten kann, wenn man die Herstellung der Salicylsäureverbindungen durch Umsetzen von Salicylsäure mit einer Styrolverbindung im molaren Verhältnis von 1:2 in Gegenwart einer aromatischen Sulfonsäure durchführt.

Gegenstand vorliegender Erfindung ist daher ein Verfahren zur Herstellung eines Gemisches aus einem Metallsalz der Formel

(1) 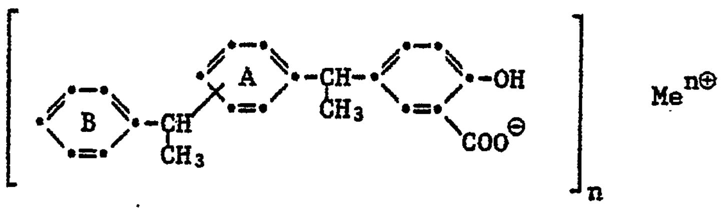

und einem Metallsalz der Formel

(2) 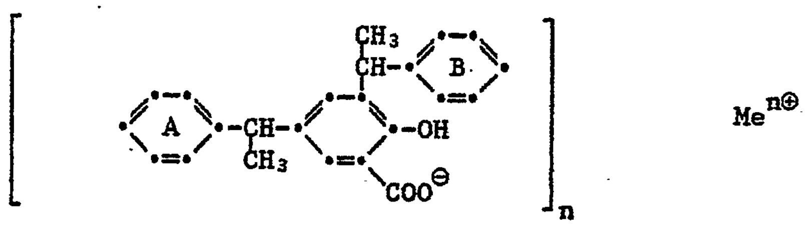

wobei in den Formeln (1) und (2)
Me ein n-wertiges Metallion und
n 2, 3 oder 4 bedeuten und
die Ringe A und B, unabhängig voneinander, unsubstituiert oder durch Halogen, Niederalkyl, Niederalkoxy oder einen α-Methylbenzylrest substituiert sind.

Das Verfahren ist dadurch gekennzeichnet, dass man 2 Mol Salicylsäure mit mindestens 2 Mol einer Styrolverbindung der Formel

(3) 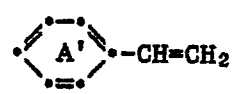

und mindestens 2 Mol einer Styrolverbindung der Formel

(4) 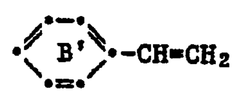

worin die Benzolringe A' und B' unsubstituiert oder durch Halogen, Niederalkyl oder Niederalkoxy substituiert sind, in Gegenwart einer aromatischen Sulfonsäure umsetzt und 2n Mol des erhaltenen Gemisches aus der Salicylsäureverbindung der Formel

(5) 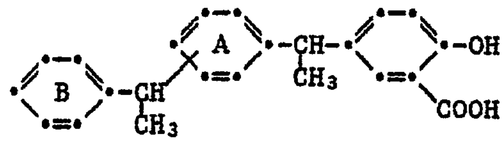

und der Salicylsäureverbindung der Formel

(6) 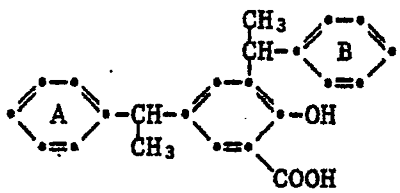

wobei in den Formeln (5) und (6) A und B die für Formeln (1) und (2) angegebenen Bedeutungen haben, mit 2 Mol des Salzes eines n-wertigen Metalls einer anorganischen Säure oder einer niederen aliphatischen Carbonsäure weiter umsetzt, wobei n die angegebene Bedeutung hat.

Die erfindungsgemäss hergestellten Metallsalzgemische können in einem Gewichtsverhältnis von 9:1 bis 1:9 vorliegen.

Die bei den Metallsalzen der Formeln (1) und (2) zweifach bis vierfach vorkommenden Salicylatbestandteile können gleich oder verschieden sein. Vorzugsweise sind sie jeweils identisch.

Niederalkyl und Niederalkoxy stellen in der Regel Gruppen dar, die 1 bis 5, insbesondere 1 bis 3 Kohlenstoffatome aufweisen, wie z.B. Methyl, Ethyl, Isopropyl, sek.-Butyl, tert.-Butyl, Amyl oder Isoamyl bzw. Methoxy, Ethoxy, Isopropoxy, n-Butoxy oder tert.-Butoxy.

Halogen bedeutet beispielsweise Fluor, Jod, Brom oder vorzugsweise Chlor.

Die erfindungsgemäss hergestellten Metallsalze sich vorteilhafterweise von 2-, 3- oder 4-wertigen Metallen mit einem Atomgewicht von 24 bis 210, vorzugsweise 26 bis 120 ab. Beispiele derartiger Metalle sind Aluminium, Barium, Blei, Cadmium, Calcium, Chrom, Eisen, Gallium, Kobalt, Kupfer, Magnesium, Mangan, Molybdän, Nickel, Quecksilber, Silber, Strontium, Tantal, Titan, Vanadium, Wolfram, Zink, Zinn und Zirkonium. Dabei sind Aluminium, Zirkonium, Vanadium, Zinn und insbesondere Zink bevorzugt. Die

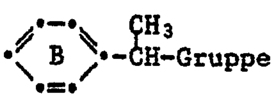

der Verbindungen der Formeln (1) und (5) befindet sich vorteilhafterweise in p-Stellung zur Ethylidengruppe.

Die Ringe A und B sind vorzugsweise nicht weiter substituiert. Falls die Ringe A und B Substituenten aufweisen, sind sie in erster Linie durch Halogen, Methyl, Methoxy oder α-Methylbenzyl weiter substituiert. Pro Benzolring A oder B können vorteilhafterweise 1 oder 2 Substituenten vorhanden sein. Der α-Methylbenzylrest ist in der Regel im Benzolring B vorhanden.

Praktisch wichtige Metallsalzgemische, die erfindungsgemäss hergestellt und vorteilhaft als Farbentwickler verwendet werden, sind Aluminiumsalze oder noch bevorzugter Zinksalze der substituierten Salicylsäureverbindungen der Formeln (5) und (6), in denen der entsprechende Ring B unsubstituiert ist.

Im Vordergrund des Interesses steht als Farbentwickler das Gemisch aus dem Zinksalz der 5-[α-Methyl-4'-(α-methylbenzyl)-benzyl-]salicylsäure und dem Zinksalz der 3,5-Bis-[α-methylbenzyl-]salicylsäure.

Die Herstellung des Gemisches aus den freien Salicylsäureverbindungen der Formeln (5) und (6) erfolgt vorteilhafterweise bei einer Temperatur zwischen 20°C und der Rückflusstemperatur, vorzugsweise bei 80°C bis 150°C.

Die Reaktionsdauer hängt in der Regel von der Temperatur des Reaktionsmediums ab und liegt zweckmässigerweise zwischen 1/2 und 5 Stunden, vorzugsweise 1 bis 3 Stunden.

Vorzugsweise sind die Styrolkomponenten der Formeln (3) und (4) identisch.

Als Beispiele für geeignete Styrolkomponenten seien genannt: Styrol, p-Chlorstyrol, p-Methylstyrol, 2,4-Dimethylstyrol.

Als aromatische Sulfonsäuren, die als Katalysatoren dienen, kommen beispielsweise Benzolsulfonsäure, Chlorbenzolsulfonsäure, Toluolsulfonsäure, Chlortoluolsulfonsäure oder Xylolsulfonsäure in Betracht. p-Toluolsulfonsäure ist bevorzugt.

Die aromatische Sulfonsäure insbesondere die Toluolsulfonsäure kann in situ hergestellt werden. Die Herstellung der Toluolsulfonsäure in situ erfolgt z.B. wie in Chemical Abstract, Vol. 79 (1969), 106228r beschrieben.

Bei Verwendung von in situ hergestellter aromatischer Sulfonsäure wird die Umsetzung der Salicylsäure mit den Styrolverbindungen der Formeln (3) und (4) vorteilhafterweise in einem nicht an der Kondensation teilnehmenden organischen Lösungsmittel durchgeführt.

Geeignete organische Lösungsmittel, die das Reaktionsmedium bilden, sind cycloaliphatische oder vorzugsweise aromatische Kohlenwasserstoffe, wie z.B. Cyclohexan, Benzol, Toluol oder Xylol; Chlorkohlenwasserstoffe, wie z.B. Ethylenchlorid, Tetrachlorethylen oder Chlorbenzole, wie z.B. Chlorbenzol, Chlortoluol oder Dichlorbenzol; cyclische Ether, wie z.B. Dioxan oder Tetrahydrofuran; Dimethylsulfoxid oder Nitrile aliphatischer Monocarbonsäuren, wie z.B. Acetonitril, Propionitril oder Butyronitril. Auch Mischungen der genannten Lösungsmittel können verwendet werden. Bevorzugte Lösungsmittel sind Chlorbenzol, Chlortoluol und besonders Toluol.

Bei Verwendung einer bereits vorbereiteten aromatischen Sulfonsäure ist das Lösungsmittel nicht erforderlich.

Das erhaltene Gemisch von den freien Salicylsäureverbindungen kann direkt zur Herstellung der Metallsalze der Formeln (1) und (2) weiterverwendet werden.

Falls eine Isolierung der substituierten Salicylsäureverbindungen der Formeln (5) und (6) erwünscht ist, wird z.B. die saure Lösung des Umsetzungsproduktes zuerst mit einer wässerigen Natriumhydroxydlösung neutralisiert und dann die neutrale Lösung mit einer niederen Carbonsäure oder einer anorganischen Säure angesäuert, worauf das Produkt in Form eines Oels ausfällt und abgetrennt wird und dann die einzelnen Säuren chromatographisch voneinander getrennt werden können.

Die Metallisierung des Salicylsäuregemisches erfolgt vorteilhafterweise in einer alkalischen Lösung der verwendeten Salicylsäureverbindungen und vorzugsweise in Gegenwart einer Alkaliverbindung, wie z.B. Alkalimetallhydroxide, -carbonate oder -bicarbonate oder Ammoniumhydroxid, Ammoniumcarbonat oder Ammoniumhydrogencarbonat.

Die Metallisierung kann schon bei einer Temperatur von 10 bis 25°C vorgenommen werden. In gewissen Fällen und besonders bei Verwendung von organischen Aluminiumsalzen ist es nötig bei höheren Temperaturen, vorzugsweise 70 bis 200°C zu arbeiten.

Mann kann die Reaktionspartner aber auch in einer Schmelze umsetzen. Als Schmelzmittel eignen sich z.B. Salze niederer Fettsäuren, wie z.B. Natriumacetat, Amide niederer Fettsäuren, wie z.B. Acetamid, ferner Harnstoff oder Thioharnstoff oder deren N-Substitutionsprodukte.

Als Metallabgebende Mittel verwendet man zweckmässig die Metallsalze von Mineralsäuren oder Carbonsäuren mit 1 bis 6 Kohlenstoffatomen, insbesondere Sulfate, Halogenide (Chloride), Nitrate, Formiate, Acetate, Propionate, Oxalate oder Citrate.

Als Beispiele für anorganische Metallsalze seien die Zinksalze Zinkchlorid, Zinksulfat oder Zinknitrat sowie auch Aluminiumsulfat, Zinndichlorid, Zirkonoxychlorid genannt.

Organische Metallsalze sind z.B. Zinkdiacetat, Zinkoxalat, Aluminiumtriisopropylat oder Aluminium-sek.butylat.

Anstelle der genannten Zinksalze können auch Zinkoxid oder Zinkcarbonat verwendet werden, wobei in diesem Falle die Umsetzung mit dem Salicylsäuregemisch, vorzugsweise in Gegenwart von Ammoniumformiat durchgeführt wird.

Eine besonders zweckmässige Ausführungsform zur Herstellung der Metall-Verbindungen der Formeln (1) und (2), in denen Me das Zinkion ist, besteht darin, dass man zu einem Reaktionsmedium bestehend aus etwa 1 Mol, vorzugsweise 1,0 bis 1,2 Mol Salicylsäure und p-Toluolsulfonsäure bei einer Temperatur von 100 bis 170°C vorzugsweise 120–160°C portionsweise Styrol zugibt und während 1 bis 3 Stunden kondensiert. Darauf wird wässerige Natriumhydroxidlösung zugegeben und die alkalische Lösung mit einem anorganischen Zinksalz, vorzugsweise Zinkchlorid behandelt, worauf das erhaltene Gemisch aus den Zinksalzen der entsprechenden Salicylsäureverbindungen isoliert wird.

Die erfindungsgemäss hergestellten Metallsalzgemische sind praktisch farb- und geruchlos und eignen sich vor allem als Farbentwickler für Farbbildner. Sie sind mit den üblichen Farbbildnern sehr reaktiv, sodass damit spontane, beständige und nicht verblassende Aufzeichnungen oder Kopien erhalten werden können. Die erfindungsgemäss hergestellten Metallsalzgemische werden vorzugsweise als Farbentwickler für Aufzeichnungsmaterialien verwendet, die sowohl Kopier- als auch Registriermaterialien sein können. Das Aufzeichnungsmaterial kann druckempfindlich oder wärmeempfindlich sein.

Die im Aufzeichnungsmaterial in Betracht kommenden Farbbildner sind bekannte farblose oder schwach gefärbte Stoffe, die, sofern sie mit dem Gemisch aus den Metallsalzen der Formeln (1) und (2) in Kontakt kommen, farbig werden oder die Farbe ändern. Farbbildner oder deren Mischungen können verwendet werden, die z.B. den Klassen der Phthalide, Azaphthalide, Fluorane, Spiropyrane, Spirodipyrane, Azomethine, Chinazoline, Leukoauramine, Triarylmethan-leukofarbstoffe, Carbazolylmethane, Rhodaminlaktame, Chromenopyrazole, Phenoxazine, Phenothiazine, sowie der Chromeno- oder Chromanfarbbildner angehören. Als Beispiele solcher geeigneten Farbbildner seien genannt Kristallviolettlacton, 3.3-(Bisaminophenyl)-phthalide, 3,3-(Bis-substituierte-indolyl)-phthalide, 3-(Aminophenyl)-3-indolyl-phthalide, 3-(Aminophenyl)-3-indolylazaphthalide, 6-Dialkylamino-2-n-octyl-amino-fluorane, 6-Dialkylamino-2-arylamino-fluorane, z.B. 6-Diethylamino-2-(2'-chlorphenylamino)-fluoran, 6-Dibutylamino-2-(2'-chlorphenylamino)-fluoran, 6-Dialkylamino-3-methyl-2-arylamino-fluorane, z.B. 2-Anilino-3-methyl-6-diethylaminofluoran oder 2-(2',4'-Dimethylanilino)-3-methyl-6-diethylaminofluoran, 6-Dialkylamino-2- oder -3-niederalkyl-fluorane, 6-Dialkylamino-2-dibenzylamino-fluorane, 6-Pyrrolidino-2-diben-

zylaminofluoran, 6-N-Cyclohexyl-N-niederalkylamino-3-methyl-2-arylamino-fluorane, 6-Pyrrolidino-2-arylamino-fluorane, Bis-(aminophenyl)-furyl- oder -phenyl-oder -carbazolyl-methane, 3'-Phenyl-7-dialkylamino-2,2'-spirodibenzopyrane, Bisdialkylamino-benzhydrol-alkyl- oder -aryl-sulfinate, Benzoyldialkylamino-phenothiazine oder -phenoxazine.

In den folgenden Herstellungs- und Anwendungsbeispielen beziehen sich die angegebenen Prozentsätze, wenn nichts anderes angegeben ist, auf das Gewicht und Teile sind Gewichtsteile.

Herstellungsbeispiele

Beispiel 1

414 g Salicylsäure und 36 g p-Toluolsulfonsäure•1H@O werden unter Rühren auf 130°C aufgeheizt, wobei sich eine partielle Schmelze bildet. Alsdann lässt man im Verlaufe von 15 Minuten 90 g Styrol zutropfen, wobei sich die Temperatur auf 137°C erhöht und eine braune klare Lösung entsteht. Man lässt im Verlaufe von 45 Minuten weitere 535 g Styrol zutropfen, wobei die Reaktionstemperatur innerhalb 10 Minuten auf 157°C ansteigt und durch Regulierung der Zutropfgeschwindigkeit bei 150–157°C gehalten wird. Nach beendigtem Zutropfen lässt man das Reaktionsgemisch noch 1 Stunde bei 155–157°C nachreagieren und anschliessend wird auf 90°C abgekühlt. Hierauf werden 1595 ml 2n-wässrige Natriumhydroxidlösung zugegeben, worauf die nach 2 Stunden abgeschiedene ölige Phase des unreagierten Styrols abgetrennt wird. Die wässrige Phase lässt man unter Rühren zu einer Lösung von 260 g Zinkchlorid in 5 Liter Wasser bei einer Temperatur von 10–15°C in dünnem Strahl zufliessen. Das ausgeschiedene Zinksalz wird abfiltriert, abgepresst, in 5 Liter aufgenommen, wieder abfiltiert, abgepresst und bei 50–70°C im Vakuum getrocknet. Man erhält 1031 g eines pulverförmigen Produktes, das ein Gemisch aus dem Zinksalz der Formel

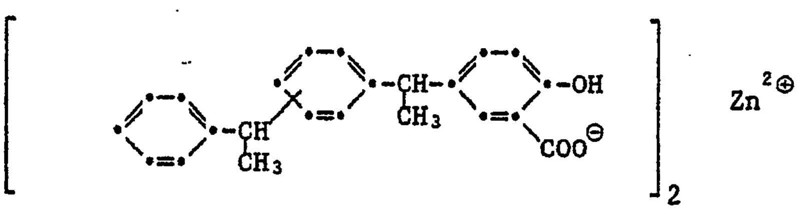
(11)

und dem Zinksalz der Formel

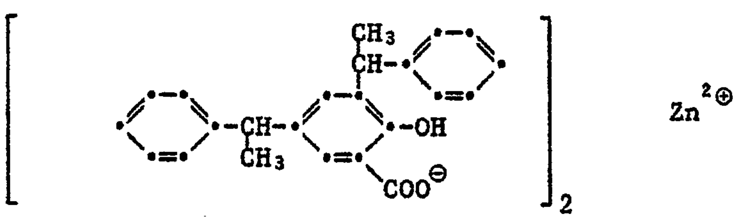
(12)

darstellt. Das NMR-Spektrum dieses Gemisches zeigt ein Gewichtsverhältnis des Gemisches von 1:1.

Das Gemisch schmilzt bei 104–118°C.

Beispiel 2

Man arbeitet analog Beispiel 1, jedoch wird die p-Toluolsulfonsäure durch 36 g Benzolsulfonsäure ersetzt. Man erhält 1074 g eines pulverförmigen Produktes, welches ein Gemisch aus 30% des Zinksalzes der Formel (11) und 70% des Zinksalzes der Formel (12) darstellt. (NMR Spektrum). Das Gemisch schmilzt bei 90–150°C.

Beispiel 3

Man arbeitet analog Beispiel 1, jedoch wird die p-Toluolsulfonsäure durch 36 g 2,5-Dimethylbenzolsulfonsäure-2H@O ersetzt. Man erhält 1077 g eines pulverförmigen Produktes, welches ein Gemisch aus 40% des Zinksalzes der Formel (11) und 60% des Zinksalzes der Formel (12) darstellt. (NMR Spektrum). Das Gemisch schmilzt bei 60–110°C.

Beispiel 4

a) 69 g Salicylsäure und 6 g p-Toluolsulfonsäure-1H@O werden unter Rühren auf 130°C aufgeheizt, wobei sich eine partielle Schmelze bildet. Alsdann lässt man im Verlaufe von 10 Minuten 15 g Styrol zutropfen, wobei sich die Temperatur auf 135°C erhöht und eine braune klare Lösung entsteht. Man lässt im Verlaufe von 35 Minuten weitere 89,2 g Styrol zutropfen, wobei die Reaktionstemperatur innerhalb 10 Minuten auf 155°C aufsteigt und durch Regulierung der Zutropfgeschwindigkeit bei 150–155°C gehalten wird. Nach beendigtem Zutropfen lässt man das Reaktionsgemisch noch 1 Stunde bei 155°C nachreagieren und anschliessend wird auf 90°C abgekühlt.

Hierauf werden 265 ml 2n-wässrige Natriumhydroxidlösung zugegeben und auf 25°C abgekühlt.

Man erhält 520 g einer braunen, klaren Lösung, welche einen Gehalt von 33 Gew.-% eines 1:1 Gemisches des Natriumsalzes der Salicyclsäureverbindungen der Formeln (13) und (14) enthält.

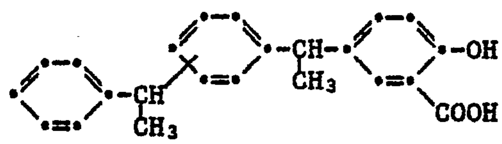

(13)

und

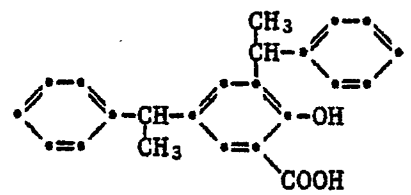

(14)

b) Zu einer Lösung von 15,7 g Zirkonoxychlorid (ZrOCl@•6H@O) in 150 g Wasser lässt man bei 20°C unter Rühren 111,5 g der gemäss a) hergestellten Na-Salzlösung zutropfen. Man erhält zunächst nur eine mässige Fällung des Zr-Salzes, welche jedoch durch weiteres Zutropfen von 250 g Wasser praktisch komplett wird. Das ausgefällte Zirkonsalz wird abfiltriert, nochmals in 250 g Wasser aufgeschlämmt, wieder abfiltriert und in feuchtem Zustand in 100 ml Toluol suspendiert. Durch azeotrope Entwässerung unter Vakuum bei 95°C erhält man das Zr-Salz mit einem H@O Gehalt von 0,9% Ausbeute: 38 g (ca: 95% d.Th.) graues Pulver; Fp. ~100–140°C.

Analyse: Ber. Zr = 11,43%, Gef. Zr = 10,7%.

Das Produkt ist das Zirkonsalz eines 1:1 Gemisches der Verbindungen der Formeln (13) und (14).

Beispiel 5

111,5 g der gemäss Beispiel 4a) hergestellten Na-Salzlösung werden im Vakuum bei 60°C zur Trockene eingedampft. Das entwässerte Salz wird bei 20°C in 100 ml Methanol gelöst. Diese Lösung lässt man bei 20°C zu einer Lösung von 10,65 g Zinndichlorid in 100 ml Methanol unter Rühren zutropfen. Man erhält praktisch keine Ausfällung und entfernt deshalb das Methanol bei 60°C im Vakuum. Der Rückstand wird in 100 ml Toluol bei 20°C gelöst und von unlöslichen Anteilen abfiltriert. Nach dem Entfernen des Toluols bei 95°C unter Vakuum erhält man 32,8 g (81% d.Th.) Zinnsalz, welches bei 0°C ein beigefarbiges Pulver ist, und >30°C in einem halbfesten Zustand vorliegt (Fp. ca. 30–50°C).

Analyse: Ber. Sn = 14,66%, Gef. Sn = 15,4%.

Das Produkt ist das Zinnsalz eines ca. 1:1 Gemisches der Verbindungen der Formeln (13) und (14).

Beispiel 6

Zu einer Lösung von, 13,3 g Aluminiumsulfat Al@ (SO$)#•18H@O in 100 g Wasser lässt man bei 20°C unter Rühren 111,5 g der gemäss Beispiel 4a) hergestellten Na-Salzlösung zutropfen. Man erhält eine deutliche Fällung des Al-Salzes, welches abfiltriert, nochmals in 150 g Wasser aufgeschlämmt und wieder abfiltriert wird. Das feuchte Produkt wird in 250 ml Toluol gelöst. Durch azeotrope Entwässerung unter Vakuum bei 95°C erhält man das Al-Salz mit einem H@O-Gehalt von 2,9%.

Ausbeute: 33,6 g (ca. 92% d.Th. ber. auf wasserfreies Produkt) beigefarbiges Pulver; Fp ~60–85°C;

Analyse: Ber. Al = 2,54%, Gef. Al = 2,13%.

Das Produkt ist das Aluminiumsalz eines 1:1 Gemisches der Salicylsäure-Verbindungen der Formeln (13) und (14).

Anwendungsbeispiele

Beispiel 1

(Druckempfindliches Aufzeichnungssystem)

Eine feingemahlene wässrige Dispersion (2–4 μm), die einen 38%igen Feststoffgehalt bestehend aus 1 Teil des Zinksalzgemisches gemäss Herstellungsbeispiel 1,

7,4 Teilen China Clay,
0,8 Teilen eines Kondensationsproduktes aus Naphthalinsulfonsäure und Formaldehyd und
0,9 Teilen eines Styrol/Butadiencopolymerisates (100%)
aufweist, wird auf ein Streichrohpapier mit einem Flächengewicht von 48 g/m2 mit einem Rakel aufgetragen. Auftragsgewicht trocken: 6–7 g/m2.

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

Beispiel 2

Eine feingemahlene wässrige Dispersion (2–4 µm), die einen 38%igen Feststoffgehalt bestehend aus
1 Teil des Zinksalzgemisches gemäss Herstellungsbeispiel 2,
10 Teilen China Clay und
0,6 Teilen Polyvinylalkohol
aufweist, wird auf ein Streichrohpapier mit einem Flächengewicht von 48 g/m2 mit einem Rakel aufgetragen. Auftragsgewicht trocken: 5–6 g/m2.

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

Beispiel 3

Eine feingemahlene wässrige Dispersion (2–4 µm), die einen 38%igen Feststoffgehalt bestehend aus
1 Teil des Zirkonsalzgemisches gemäss Herstellungsbeispiel 4,
10 Teilen China Clay und
0,6 Teilen Polyvinylalkohol
aufweist, wird auf ein Streichrohpapier mit einem Flächengewicht von 48 g/m2 mit einem Rakel aufgetragen. Auftragsgewicht trocken: 5–6 g/m2.

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

Beispiel 4

Eine feingemahlene wässrige Dispersion (2–4 µm), die einen 38%igen Feststoffgehalt bestehend aus
1 Teil des Aluminiumsalzgemisches gemäss Herstellungsbeispiel 6,
10 Teilen China Clay und
0,6 Teilen Polyvinylalkohol
aufweist, wird auf ein Streichrohpapier mit einem Flächengewicht von 48 g/m2 mit einem Rakel aufgetragen. Auftragsgewicht trocken: 5–6 g/m2.

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

Beispiel 5

0,6 Teile des Zinnsalzgemisches gemäss Herstellungsbeispiel 5 werden in 8 Teilen Toluol gelöst und auf einem Streichrohpapier von 1 m2 Fläche (Gewicht: 48 g/m2) aufgetragen. Auftragsgewicht trocken: ca. 0,6 g/m2.

Die so hergestellte Nehmerschicht wird mit der Geberschicht eines handelsüblichen Durchschreibepapiers (z.B. Zanders) benachbart aufeinandergelegt. Die Geberschicht enthält in Mikrokapseln gelöst den Farbbildner, z.B. Kristallviolettlakton. Nach Durchschrift mit der Hand oder Schreibmaschine entsteht eine intensiv blaue Kopie.

## Patentansprüche

1. Verfahren zur Herstellung eines Gemisches aus einem Metallsalz der Formel

(1) 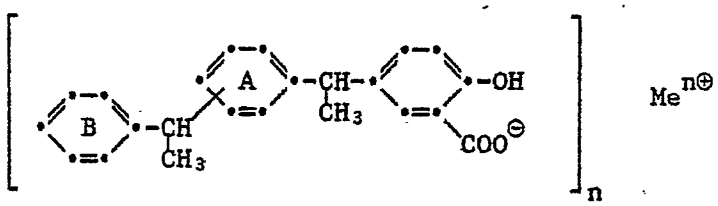

und einem Metallsalz der Formel

(2) 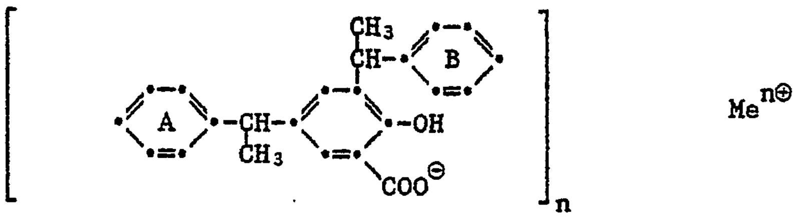

wobei in den Formeln (1) und (2)
Me ein n-wertiges Metallion und
n 2, 3 oder 4 bedeuten und
die Ringe A und B, unabhängig voneinander, unsubstituiert oder durch Halogen, Niederalkyl, Niederalkoxy oder einen α-Methylbenzylrest substituiert sind, dadurch gekennzeichnet, dass man 2 Mol Salicylsäure mit mindestens 2 Mol einer Styrolverbindung der Formel

(3) 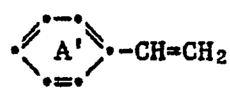

und mindestens 2 Mol einer Styrolverbindung der Formel

(4) 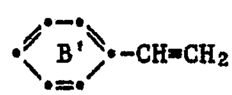

worin die Benzolringe A' und B' unsubstituiert oder durch Halogen, Niederalkyl oder Niederalkoxy substiuiert sind, in Gegenwart einer aromatischen Sulfonsäure umsetzt und 2n Mol des erhaltenen Gemisches aus der Salicylsäureverbindung der Formel

(5) 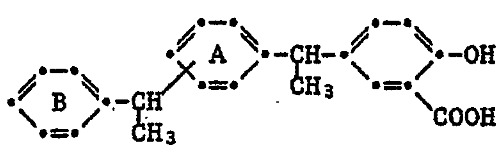

und der Salicylsäureverbindung der Formel

(6) 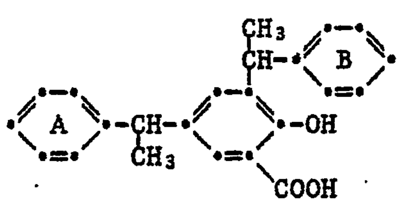

wobei in den Formeln (5) und (6) A und B die für Formeln (1) und (2) angegebenen Bedeutungen haben, mit 2 Mol des Salzes eines n-wertigen Metalls einer anorganischen Säure oder einer niederen aliphatischen

Carbonsäure weiter umsetzt, wobei n die angegebene Bedeutung hat.

2. Verfahren gemäss Anspruch 1, dadurch gekennzeichnet, dass in den Formeln (1) und (2) Me das Aluminium-, Vanadium-, Zinn-, Zirkon- oder vorzugsweise Zinkion ist.

3. Verfahren gemäss einem der Ansprüche 1 und 2, dadurch gekennzeichnet, dass in den Formeln (1) und (2) die Ringe A und B unsubstituiert sind.

4. Verfahren gemäss einem der Ansprüche 1 und 2, dadurch gekennzeichnet, dass in den Formeln (1) und (2) der Ring B unsubstituiert oder durch α-Methylbenzyl substituiert ist.

5. Verfahren gemäss einem der Ansprüche 1 bis 3, dadurch gekennzeichnet, dass das Verfahrenserzeugnis das Gemisch aus dem Zinksalz der 5-[α-Methyl-4'-(α-methylbenzyl)-benzyl-]salicylsäure und dem Zinksalz der 3,5-Bis-[α-methylbenzyl-]salicylsäure ist.

6. Verfahren gemäss einem der Ansprüche 1 bis 5, dadurch gekennzeichnet, dass das hergestellte Gemisch in einem Gewichtsverhältnis der Metallsalzkomponenten von 1:9 bis 9:1 liegt.

7. Verfahren gemäss einem der Ansprüche 1 bis 6, dadurch gekennzeichnet, dass die aromatische Sulfonsäure p-Toluolsulfonsäure ist.

8. Verfahren gemäss einem der Ansprüche 1 bis 6, dadurch gekennzeichnet, dass als aromatische Sulfonsäure in situ hergestellte Toluolsulfonsäure verwendet wird.

**Claims**

1. A process for the preparation of a mixture of a metal salt of formula

(1) 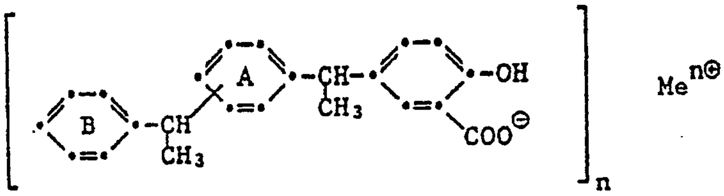

and a metal salt of formula

(2) 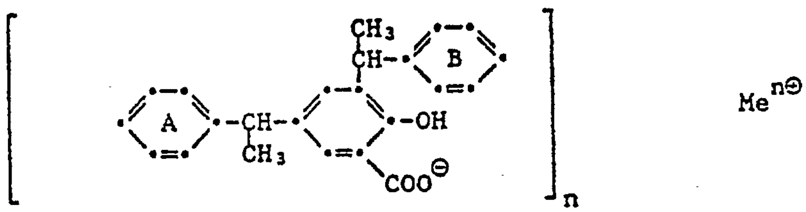

in which formulae (1) and (2)
Me is an n-valent metal ion,
n is 2, 3 or 4, and
each of the rings A and B independently of the other is unsubstituted or substituted by halogen, lower alkyl, lower alkoxy or an α-methylbenzyl radical, which process comprises reacting 2 moles of salicylic acid with at least 2 moles of a styrene compound of formula

(3) 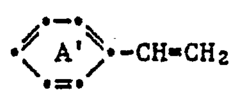

and at least 2 moles of a styrene compound of formula

(4) 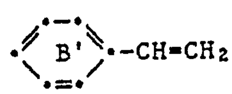 ,

in which the benzene rings A' and B' are unsubstituted or substituted by halogen, lower alkyl or lower alkoxy, in the presence of an aromatic sulfonic acid, and, in a further step, reacting 2n moles of the resultant mixture of the salicylic acid compound of formula

(5) 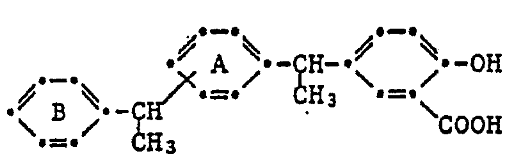

and the salicylic acid compound of formula

(6) 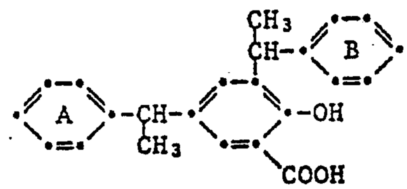

in which formulae (5) and (6) A and B are as defined for formulae (1) and (2), with 2 moles of the salt of an n-valent metal of an inorganic acid or of a lower aliphatic carboxylic acid, where n has the given meaning.

2. A process according to claim 1, wherein, in formulae (1) and (2). Me is the aluminium, vanadium, tin, zirconium or preferably the zinc ion.

3. A process according to either of claims 1 and 2, wherein, in formulae (1) and (2), the rings A and B are unsubstituted.

4. A process according to either of claim 1 and 2, wherein, in formulae (1) and (2), the ring B is unsubstituted or substituted by α-methylbenzyl.

5. A process according to any one of claims 1 to 3, wherein the product is a mixture of the zinc salt of 5-[αmethyl-4'-(methylbenzyl)benzyl]-salicylic acid and the zinc salt of 3,5-bis-[α-ethylbenzyl]salicylic acid.

6. A process according to any one of claims 1 to 5, wherein the metal salt components of the mixture prepared are in a weight ratio of 1:9 to 9:1.

7. A process according to any one of claims 1 to 6, wherein the aromatic sulfonic acid is p-toluenesulfonic acid.

8. A process according to any one of claims 1 to 6, wherein the aromatic sulfonic acid used is toluenesulfonoc acid which is prepared in situ.

**Revendications**

1. Procédé pour la préparation d'un mélange d'un sel métallique de formule

(1) 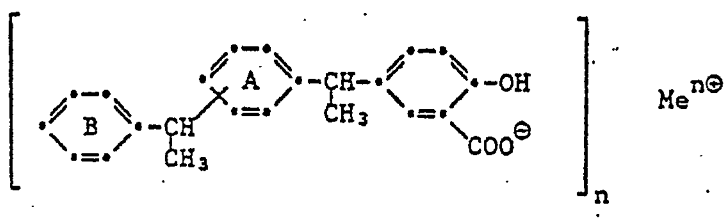

et d'un sel mètallique de formule

(2) 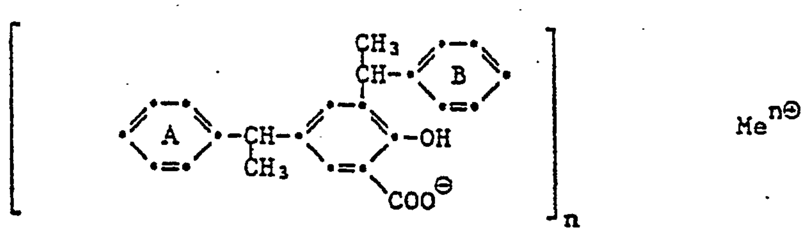

où, dans les formules (1) et (2):

Me est un ion métallique n-valent et

n vaut 2, 3 ou 4, et

les noyaux A et B, indépendamment l'un de l'autre, sont non-substitués, ou substitués par un radical halogéno, alkyle inférieur ou elcoxy inférieur, ou par un radical α-méthylbenzyle, caractérisé en ce qu'on fait réagir, en présence d'un acide sulfonique aromatique, 2 moles d'acide salicylique sur au moins 2 moles d'un composé du styrène, de formule

(3) 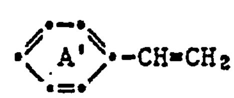

et sur au moins 2 moles d'un composé du styrène de formule

(4) 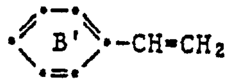 ,

où les noyaux benzéniques A' et B' sont non-substitués, ou substitués par un radical halogéno, alkyle inférieur ou alcoxy inférieur, puis on fait réagir, sur 2 moles du sel d'un métal n-valent d'un acide inorganique ou d'un acide carboxylique aliphatique inférieur, 2 n moles du mélange obtenu, constitué du composé de l'acide salicylique de formule

(5) 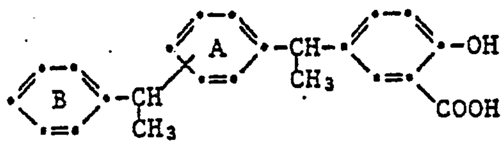

et du composé de l'acide salicylique de formule

(6) 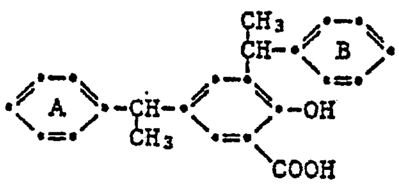

où, dans les formules (5) et (6), A et B ont les significations données pour les formules (1) et (2), n ayant la signification indiquée.

2. Procédé selon la revendication 1, caractérisé en ce que, dans les formules (1) et (2), Me est l'ion aluminium, vanadium, étain, zirconium ou de préférence zinc.

3. Procédé selon l'une des revendications 1 ou 2, caractérisé en ce que, dans les formules (1) et (2), les noyaux A et B sont non-substitués.

4. Procédé selon l'une des revendications 1 ou 2, caractérisé en ce que, dans les formules (1) et (2), le noyau B est non-substitué, ou substitué par un radical α-méthylbenzyle.

5. Procédé selon l'une des revendications 1 à 3, caractérisé en ce que le produit obtenu est le mélange du sel de zinc de l'acide 5-[α-méthyl-4'-(α-éthyl-benzyl)-benzyl-]salicylique et du sel de zinc de l'acide 3,5-bis-[α-méthylbenzyl-]salicylique.

6. Procédé selon l'une des revendincations 1 à 5, caractérisé en ce que, dans le mélange obtenu, le rapport pondéral entre les composants sels métalliques est compris entre 1:9 et 9:1.

7. Procédé selon l'une des revendications 1 à 6, caractérisé en ce que l'acide sulfonique aromatique est l'acide p-toluènesulfonique.

8. Procédé selon l'une des revendications 1 à 6, caractérisé en ce qu'on utilise comme acide sulfonique aromatique de l'acide toluènesulfonique préparé in situ.